# EUROPEAN PATENT APPLICATION

(11) **EP 4 524 129 A1**
(43) Date of publication of application: **19.03.2025**
(21) Application number: 22941031.1
(22) Date of filing: 09.05.2022
(51) Int. Cl.: C07C 253/30, C07C 255/33

(54) **METHOD FOR PREPARING 2-SUBSTITUTED ARYL ACETONITRILE COMPOUND AND USE THEREOF**

(71) Applicant: Maxunitech Inc., Hangzhou, Zhejiang 310052 (CN)
(72) Inventor: CHEN, Bangchi, Hangzhou, Zhejiang 310052 (CN); SUN, Yinwei, Hangzhou, Zhejiang 310052 (CN); WANG, Zhongyuan, Hangzhou, Zhejiang 310052 (CN); ZHONG, Yi, Hangzhou, Zhejiang 310052 (CN)
(74) Representative: Haseltine Lake Kempner LLP
(86) International application number: PCT/CN2022/091714
(87) International publication number: WO 2023/216063

(57) **Abstract**

A method for preparing 2-substituted arylacetonitrile compound of formula (1) and an use thereof. In this method, a substituted aromatic compound of formula (2) and a 2-cyanocarboxylate compound of formula (3) are directly reacted in the presence of a base to obtain the 2-substituted arylacetonitrile compound (1), as shown in the following reaction scheme: 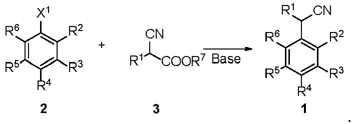 This method has high yield, and does not require the transition metal catalyst and strong base, and thus can be carried out in a mild reaction condition.

## Description

### TECHNICAL FIELD

This application relates to organic synthesis, and more particularly to a method for preparing a 2-substituted arylacetonitrile compound and an use thereof.

### BACKGROUND

2-Substituted arylacetonitriles are a class of important organic compounds. They can be transformed to aryl ketones through oxidation reactions (J. Org. Chem. 1983,48,4087-4096). Aryl ketones are key intermediates in preparation of many important agricultural fungicides. For example, 1-(4-chlorophenyl)-2-cyclopropylpropan-1-one is a key intermediate in preparation of the triazole fungicide cyproconazol (CN 103044230). 4-(4-Chlorophenoxy)-2-(trifluoromethyl)phenylethanone is a key intermediate in preparation of fungicide mefentrifluconazole (CN 105152899). Therefore, it is of great significance to provide a simple and efficient method to prepare 2-substituted arylacetonitrile compounds.

The first synthetic method for 2-substituted arylacetonitrile compounds is performed through alkylation reaction of arylacetonitriles. The synthesis of the starting material arylacetonitriles in this method usually uses highly toxic cyanide, such as potassium cyanide. In addition, such method is easy to stop at the desired monoalkylated product, and the reaction may continuously occur to obtain a dialkylated product. For example, WO 2016037578 reported that the main product is 2,2-dimethyl-(2'-trifluoromethyl) phenylacetonitrile in the reaction of 2-(trifluoromethyl)phenylacetonitrile with iodomethane in the presence of sodium hydride, shown as Reaction Scheme 1:

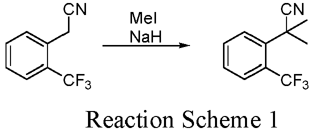

CN105753685 disclosed a second method for the preparation of the 2-substituted arylacetonitrile compounds. This method uses ethyl 2-cyano-2-phenylacetate as starting material. Methyl 2-cyano-2-phenylacetate 2-cyano-2-phenylacetate undergoes first methylation reaction using dimethyl sulfate, and then decarboxylation in the presence of sodium methoxide to provide product, shown as Reaction Scheme 2: (this method has a complicated process)

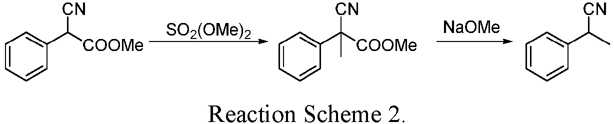

Wang et al. reported a third method for the preparation of 2-substituted arylacetonitrile compounds (Adv. Synth. Catal. 2016, 358, 940-946).This method uses 2-cyanocarboxylate as starting material. 2-Cyanocarboxylate reacts with diphenyl iodonium salt first, followed by decarboxylation in the presence of base to provide the 2-substituted arylacetonitrile compound, shown as Reaction Scheme 3. This method requires expensive diphenyl iodonium salt. Its atomic efficiency is poor, and its cost is high. It is not amenable for large scale production:

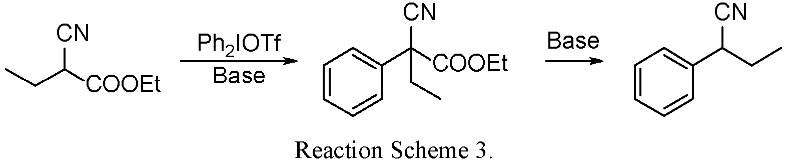

Ma et al. reported a fourth method for the preparation of 2-substituted arylacetonitrile compounds (Angew. Chem. Int. Ed. 2021, 60, 7082-7086). This method uses 2-cyanocarboxylate as starting material. 2-Cyanocarboxylate and aryl bromide undergo coupling reaction in the presence of cuprous catalyst and ligand, and subsequent decarboxylation to afford the 2-substituted arylacetonitrile compound, shown as Reaction Scheme 4. Such method avoids the shortcoming of using expensive diaryliodonium salts, however it requires transition metal catalysts, specific ligands, and strong bases such as sodium tert-butoxide, in addition to extending the reaction time up to 24 hours.

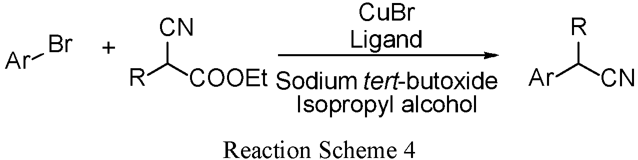

During the course of developing efficient method for the preparation of 2-substituted arylacetonitrile compounds, the inventors surprisingly discovered that 2-cyanocarboxylate and halogenated benzene or phenyl arylsulfonate compound undergo one step reaction in the presence of base to provide directly the target 2-substituted arylacetonitrile compounds in high yield, thus eliminating the use of CuBr ligand as catalyst in the aforementioned technical protocol.

### SUMMARY

This application provides a method for preparing a 2-substituted arylacetonitrile compound of formula (1), comprising:
reacting a substituted aromatic compound of formula (2), a 2-cyanocarboxylate compound of formula (3) in the presence of a base to obtain the 2-substituted arylacetonitrile compound (1), as shown in the following reaction scheme 5: 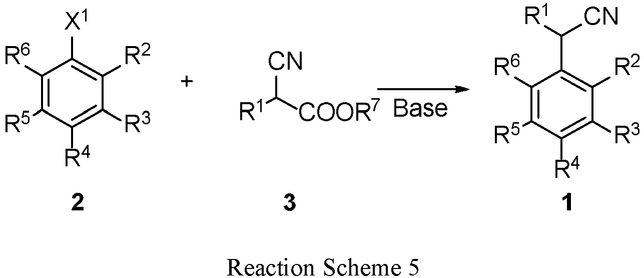
wherein R¹ is a carbon-based group.

In some embodiments, R¹ is selected from the group consisting of C₁-C₁₀ alkyl, C₆-C₁₂ aryl, and a heteroaryl group containing one or two atoms selected from nitrogen, oxygen and sulfur; wherein the alkyl, the aryl and the heteroaryl group are unsubstituted or substituted by halogen. Preferably, R¹ is C₁-C₄ alkyl, wherein the C₁-C₄ alkyl is unsubstituted or substituted by halogen.
R², R³, R⁴, R⁵, and R⁶ are independently selected from the group consisting of hydrogen, C₁-C₁₀ alkyl, C₆-C₁₂ aryl, a heteroaryl group containing one or two atoms selected from nitrogen, oxygen and sulfur, halogen, nitro, a cyano group, a C₁-C₆ alkyl-acyl group, a C₁-C₆ alkoxy group, a C₆-C₁₂ aryloxy group and -COOR⁸, wherein the alkyl, the aryl and the heteroaryl group are unsubstituted or substituted with halogen;
or, any two adjacent groups of R², R³, R⁴, R⁵, and R⁶ together form a C₁-C₁₀ cyclic substituent; the C₁-C₁₀ cyclic substituent is free of heteroatoms or containing one or two atoms selected from nitrogen, oxygen and sulfur; and the cyclic substituent is unsubstituted or substituted with halogen;
at least one of R², R⁴ and R⁶ is selected from the group consisting of a haloalkyl group, nitro, a cyano group, halogen, a C₁-C₆ alkyl-acyl group and -COOR⁸.

In some embodiments, R² and R⁴ are independently selected from the group consisting of a C₁-C₄ haloalkyl group, nitro, a cyano group, a C₂-C₄ alkyl-acyl group and -COOR⁸;
R³, R⁵, and R⁶ are independently selected from the group consisting of hydrogen and C₁-C₄ alkyl;
R⁸ is C₁-C₄ alkyl or a C₁-C₂ haloalkyl group; and
R⁷ is selected from the group consisting of hydrogen, C₁-C₁₀ alkyl, C₆-C₁₂ aryl, and a heteroaryl group containing one or two atoms selected from nitrogen, oxygen and sulfur; and the alkyl, the aryl and the heteroaryl group is unsubstituted or substituted by halogen.
R⁸ is a carbon-based group, preferably selected from the group consisting of C₁-C₁₀ alkyl, C₆-C₁₂ aryl, and a heteroaryl group containing one or two atoms selected from nitrogen, oxygen and sulfur, wherein the alkyl, the aryl, and the heteroaryl group are unsubstituted or substituted by halogen. Preferably, R⁸ is C₁-C₄ alkyl or a C₁-C₂ haloalkyl group.
X¹ is halogen or -OSO₂R⁹; wherein R⁹ is selected from the group consisting of C₁-C₁₀ alkyl, C₆-C₁₂ aryl, and a heteroaryl group containing one or two atoms selected from nitrogen, oxygen and sulfur, wherein the alkyl, the aryl and the heteroaryl group is unsubstituted or substituted by halogen; and
the base is selected from the group consisting of an alkali metal carbonate, an alkali metal bicarbonate, an alkali metal phosphate, an alkali metal hydroxide, an alkali metal alcoholate, an alkali metal hydride, an alkali metal C₁-C₆ alkyl carboxylate, an alkali metal formate, an alkaline earth metal carbonate, an alkaline earth metal bicarbonate, an alkaline earth metal phosphate, an alkaline earth metal hydroxide, an alkaline earth metal alcoholate, an alkaline earth metal hydride, an alkaline earth metal C₁-C₆ alkyl carboxylate, an alkaline earth metal formate, an alkali metal alkylide, an alkali metal amide, an organic amine compound, and a combination thereof.

In some embodiments, the base is selected from the group consisting of an alkali metal carbonate, an alkali metal bicarbonate, an alkali metal phosphate, an alkali metal hydroxide, an alkali metal C₁-C₆ alkyl carboxylate, an alkali metal formate, a tertiary amine compound, and a combination thereof; and the tertiary amine compound comprises a substituted or unsubstituted pyridine compound.

In some embodiments, the base is selected from the group consisting of potassium carbonate, potassium hydroxide, sodium carbonate, sodium hydroxide, an alkyl tertiary amine compound (comprising a cyclic tertiary alkylamine compound) and a combination thereof; wherein alkyl is a C₁-C₁₂ alkyl, preferably a C₁-C₆ alkyl.

In some embodiments, a molar ratio of the compound (3) to the compound (2) is (0.7-3.5):1. In some embodiments, the molar ratio of the compound (3) to the compound (2) is (1.4-2.2):1, preferably (1.6-1.8):1.

In some embodiments, the molar ratio of the compound (3) to the compound (2) is 3.5:1, 3.0:1, 2.5:1, 2.4:1, 2.3:1, 2.2:1, 2.1:1, 2.0:1, 1.9:1, 1.8:1, 1.7:1, 1.6:1, 1.5:1, 1.4:1, 1.3:1, 1.2:1, 1.1:1, 1.0:1, 0.9:1, 0.8:1, or 0.7:1.

In some embodiments, a molar ratio of the compound (3) to the base is 1:(0.3-2.0), preferably 1:(0.7-2.0), and more preferably 1:(0.9-1.1).

In some embodiments, the molar ratio of the compound (3) to the base is 1:0.3, 1:0.4, 1:0.5, 1:0.6, 1:0.7, 1:0.8, 1:0.9, 1:1.0, 1:1.1, or 1:2.0.

The reaction can be performed in the presence of a solvent. The solvent is selected from the group consisting of nitrile, ester, alkyl halide, ether, aromatic hydrocarbon, tertiary amine, amide, sulphone, sulfoxide, water, alcohol, ketone, and a combination thereof. In some embodiments, the solvent is selected from the group consisting of amide, sulphone, sulfoxide, water, and a combination thereof. Preferably, the solvent is selected from the group consisting of *N,N-*dimethylformamide, N-methylpyrrolidone, N,N-dimethylacetamide, dimethyl sulfoxide, water, and a combination thereof.

The reaction can be performed without the presence of a solvent.

The reaction is performed at -20-150°C, preferably 20-100°C, and more preferably 50-80°C.

It should be noted that unless otherwise stated, terms "comprise", "include" and any variations thereof, are intended to cover non-exclusive inclusion, for example, other elements that are not explicitly listed or inherent may also be included in addition to the series of elements listed.

Unless otherwise specified, the term "or" is non-exclusive. For example, "A or B" includes "A is true (or exists) and B is false (or does not exist)", "A is false (or does not exist) and B is true (or exists)", and "both A and B are true (or exist)".

The carbon group is a positive monovalent group including a carbon atom. The carbon group is connected to other parts of the chemical structure through a carbon atom. The carbon group can be saturated, unsaturated (including aryl), chain, cyclic (including polycyclic), and can includes heteroatoms. Although the carbon group is not limited in size, usually, it includes 1-16 carbon atoms and 0-3 heteroatoms. Key carbon group is C₁-C₆ alkyl, C₁-C₄ haloalkyl, or phenyl substituted with 1-3 substituents selected from C1-C3 alkyl, halogen and nitryl. The alkyl may be linear or branched. The halogen includes fluorine, chlorine, bromine and iodine. In addition, regarding haloalkyl, alkyl therein may be partially or completely substituted by halogen atoms which may be the same or different. For example, haloalky is F₃C, ClCH₂, CF₃CH₂ or CF₃CCl₂.

In some embodiments, the method is performed in the presence of the base and solvent. The 2-cyanocarboxylate compound (3) and the substituted aromatic compound (2) undergo substitution and decarboxylation to obtain the compound (1).

In a second aspect, this application provides a use of the method in the preparation of a triazole fungicide, where the triazole fungicide is selected from the group consisting of mefentrifluconazole, Ipfentrifluconazole, epoxiconazole, flutriafol, cyproconazole, bromuconazole, difenoconazole, propiconazole, etaconazole and hexaconazole.

Compared to the prior art, this application has the following beneficial effects.

The method has readily-available raw materials. The substitution and decarboxylation are carried out in a simplified "one-step" manner. In addition, it does not require transition metal catalysts and ligands.

The substituted aromatic compound (2) can be prepared according to the existing method, such as *Basic Organic Chemistry* (fourth edition), Sections 16.4, 18.8 and 18.9 Xing Qiyi et al. The 2-cyanocarboxylate compound (3) can be prepared according to the existing method, such as Organic & Biomolecular Chemistry (2017), 15(11), 2376-2384*.*

### DETAILED DESCRIPTION OF EMBODIMENTS

This application will be described in detail below with reference to the embodiments to make objects, technical features and advantages of this application clearer, but these embodiments are not intended to limit the scope of this application.

### Example 1 Preparation of 2-(4-nitro-2-(trifluoromethyl)phenyl)propionitrile

To a 100 mL reaction flask were sequentially added 5.1 g of ethyl 2-cyanopropionate, 5.5 g of potassium carbonate, 20 mL of *N,N-*dimethylformamide and 5 g of 2-trifluoromethyl-4-nitrochlorobenzene. The reaction mixture was heated in a 65°C oil bath, reacted at 65°C for 6 h, cooled and added into 100 mL of methylbenzene. The resultant reaction solution was washed, concentrated and purified to obtain 4.7 g of 2-(4-nitro-2-(trifluoromethyl)phenyl)propionitrile (87% yield).

¹H NMR (CDCl₃, 500 MHz, TMS): δ 8.58 (d, *J* = 2 Hz, 1H), 8.51 (dd, *J₁* = 8.5 Hz, *J₂* = 2 Hz, 1H), 8.00 (d, *J=* 8.5 Hz, 1H), 4.38 (q, *J* = 7 Hz, 1H), 1.72 (d, *J=* 7 Hz, 3H). ¹³C NMR (CDCl₃, 125 MHz): δ 147.3, 142.9, 131.0, 129.1 (q, *J* = 33 Hz), 127.6, 122.6 *(q, J= 273* Hz), 122.1 (q, *J* = 5 Hz), 119.9, 28.1, 21.9.

### Example 2 Preparation of 2-(4-nitro-2-(trifluoromethyl)phenyl)propionitrile

To a 100 mL reaction flask were sequentially added 51 g of ethyl 2-cyanopropionate, 4.5 g of triethylenediamine, 55 g of potassium carbonate, 200 mL of dimethyl sulfoxide and 50 g of 2-trifluoromethyl-4-nitrochlorobenzene. The reaction mixture was heated in an 80°C oil bath, reacted at 80°C for 5 h, desolventized under reduced pressure, and added into 200 mL of methylbenzene. The resultant reaction solution was washed, concentrated and purified to obtain 46 g of 2-(4-nitro-2-(trifluoromethyl)phenyl)propionitrile (85% yield).

### Example 3 Preparation of 2-(4-nitro-2-(trifluoromethyl)phenyl)propionitrile

To a 100 mL reaction flask were sequentially added 5.6 g of ethyl 2-cyanopropionate, 4.7 g of sodium carbonate, 5 g of 1-chloro-2-(trifluoromethyl)-4-nitrobenzene and 10 mL of N-methylpyrrolidone. The reaction mixture was heated in a 50°C oil bath, reacted at 50°C for 5 h, cooled and added into 100 mL of methylbenzene. The resultant reaction solution was washed, concentrated and purified to obtain 5 g of 2-(4-nitro-2-(trifluoromethyl)phenyl)propionitrile (92% yield).

### Example 4 Preparation of 2-(4-nitro-2-(trifluoromethyl)phenyl)propionitrile

To a 100 mL reaction flask were sequentially added 5.1 g of ethyl 2-cyanopropionate, 5.5 g of potassium carbonate, 0.2 g of copper(I) bromide (CuBr), 20 mL of N,N-dimethylformamide and 5 g of 1-chloro-2-(trifluoromethyl)-4-nitrobenzene. The reaction mixture was heated in a 65°C oil bath, reacted at 65°C for 6 h, cooled and added into 100 mL of methylbenzene. The resultant reaction solution was washed, concentrated and purified to obtain 3.1 g of 2-(4-nitro-2-(trifluoromethyl)phenyl)propionitrile (57% yield).

### Example 5 Preparation of 2-(5-methyl-2,4-dinitrophenyl)propionitrile

To a 50 mL reaction flask were sequentially added 5 mL of dimethyl sulfoxide, 0.5 g of 5-chloro-2,4-dinitrotoluene, 0.53 g of ethyl 2-cyanopropionate, and 0.38 g of triethylenediamine. The reaction mixture was heated to 60°C, reacted at 60°C for 6 h, cooled and added into 50 mL of methylbenzene. The resultant reaction solution was washed, concentrated and purified to obtain 0.41 g of 2-(5-methyl-2,4-dinitrophenyl)propionitrile (76% yield).

### Example 6 Preparation of 2-(4-acetyl-2-nitrophenyl)propiononitrile

To a 50 mL reaction flask were sequentially added 10 mL of *N,N-*dimethylformamide, 2 g of 4-chloro-3-nitroacetophenone, 2.3 g of ethyl 2-cyanopropionate and 2.5 g of potassium carbonate. The reaction mixture was heated to 60°C, reacted at 60°C for 5 h, cooled and added into 50 mL of methylbenzene. The resultant reaction solution was washed, concentrated and purified to obtain 1.7 g of 2-(4-acetyl-2-nitrophenyl)propiononitrile (78% yield).

¹H NMR (CDCl₃, 500 MHz, TMS): δ 8.58 (d, *J* = 1.5 Hz, 1H), 8.27 (dd, *J₁* = 8.5 Hz, *J₂* = 1.5 Hz, 1H), 7.95 (d, *J* = 8.5 Hz, 1H), 4.80 (q, *J* = 7 Hz, 1H), 2.69 (s, 3H), 1.75 (d, *J* = 7 Hz, 3H). ¹³C NMR (CDCl₃, 125 MHz): δ 194.8, 147.6, 137.8, 136.4, 133.2, 130.3, 125.2, 120.0, 28.2, 26.6, 21.0.

### Example 7 Preparation of 2-(1-cyanoethyl)-5-nitrobenzonitrile

To a 50 mL reaction flask were sequentially added 12 mL of *N,N-*dimethylformamide, 2 g of 2-chloro-5-nitrobenzonitrile, 2.5 g of ethyl 2-cyanopropionate and 2.7 g of potassium carbonate. The reaction mixture was heated to 60°C, reacted at 60°C for 5 h, cooled and added into 50 mL of methylbenzene. The resultant reaction solution was washed, concentrated and purified to obtain 1.66 g of 2-(1-cyanoethyl)-5-nitrobenzonitrile (76% yield).

¹H NMR (CDCl₃, 500 MHz, TMS): δ 8.58 (d, *J* = 2.5 Hz, 1H), 8.54 (dd, *J₁* = 9.0 Hz, *J₂* = 2.5 Hz, 1H), 7.98 (d, *J* = 9.0 Hz, 1H), 4.45 (q, *J* = 7.5 Hz, 1H), 1.78 (d, *J* = 7.5 Hz, 3H). ¹³C NMR (CDCl₃, 125 MHz) δ 147.5, 147.0, 129.3, 128.5, 128.4, 118.8, 114.5, 112.9, 30.3, 20.8.

### Example 8 Preparation of 2-(4-nitro-2-(trifluoromethyl)phenyl)-3-methylbutanenitrile

To a 100 mL reaction flask were sequentially added 6 g of ethyl 2-cyano-3-methylbutanoate, 5 g of 1-chloro-2-(trifluoromethyl)-4-nitrobenzene, 5 g of potassium carbonate and 20 mL of *N,N-*dimethylformamide. The reaction mixture was heated to 100°C, reacted at 100°C for 20 h, cooled and added into 100 mL of methylbenzene. The resultant reaction solution was washed, concentrated and purified to obtain 5 g of 2-(4-nitro-2-(trifluoromethyl)phenyl)-3-methylbutanenitrile (83% yield).

### Example 9 Preparation of ethyl 2-(1-cyanoethyl)-5-nitrobenzoate

To a 50 mL reaction flask were sequentially added 12 mL of dimethylsulfoxide, 2 g of ethyl 2-chloro-5-nitrobenzoate, 2.0 g of ethyl 2-cyanopropionate and 2.2 g of potassium carbonate. The reaction mixture was heated to 60°C, reacted at 60°C for 10 h, cooled and added into 50 mL of methylbenzene. The resultant reaction solution was washed, concentrated and purified to obtain 2 g of 2-(1-cyanoethylamino)-5-nitrobenzoate (92% yield).

¹H NMR (CDCl₃, 500 MHz, TMS): δ 8.84 (d, *J* = 2.5 Hz, 1H), 8.43 (dd, *J₁* = 9.0 Hz, *J₂* = 2.5 Hz, 1H), 7.94 (d, *J* = 9.0 Hz, 1H), 5.27 (q, *J* = 7.0 Hz, 1H), 4.46 (q, *J* = 7.0 Hz, 2H), 1.69 (d, J = 7.0 Hz, 3H), 1.47 (t, *J* = 7.0 Hz, 3H). ¹³C NMR (CDCl₃, 125 MHz): δ 164.4, 147.2, 145.7, 129.9, 129.3, 127.2, 126.3, 120.8, 62.4, 28.4, 21.3, 14.0.

### Example 10 Preparation of 4-(1-cyanoethyl)-3-nitrobenzotrifluoride

To a 50 mL reaction flask were sequentially added 12 mL of *N,N-*dimethylformamide, 2 g of 4-chloro-3-nitrobenzotrifluoride, 2.0 g of ethyl 2-cyanopropionate and 2.2 g of potassium carbonate. The reaction mixture was heated to 60°C, reacted at 60°C for 10 h, cooled and added into 50 mL of methylbenzene. The resultant reaction solution was washed, concentrated and purified to obtain 1.5 g of 4-(1-cyanoethylamino)-3-nitrobenzotrifluoride (70% yield).

¹H NMR (CDCl₃, 500 MHz, TMS): δ 8.33 (s, 1H), 8.01 (s, 2H), 4.82 (q, *J* = 7.0 Hz, 1H), 1.76 (d, *J* = 7.0 Hz, 3H). ¹³C NMR (CDCl₃, 125 MHz): δ 198.4, 145.6, 140.9, 132.8 (q, *J* = 34 Hz), 130.9 (q, *J* = 4 Hz), 128.3, 122.3 (q, *J =* 271 Hz), 121.7 (q, *J* = 4 Hz), 29.9.

### Example 11 Preparation of 2-(4-nitro-2-(trifluoromethyl)phenyl)-3-phenylpropanenitrile

To a 50 mL reaction flask were sequentially added 6 mL of *N,N-*dimethylformamide, 1 g of 2-chloro-5-phenylpropanenitrile, 1.6 g of ethyl 2-cyanophenylpropionate and 1.1 g of potassium carbonate. The reaction mixture was heated to 60°C, reacted at 60°C for 6 h, cooled and added into 50 mL of methylbenzene. The resultant reaction solution was washed, concentrated and purified to obtain 1.25 g of 2-(4-nitro-2-(trifluoromethyl)phenyl)-3-phenylpropanenitrile (88% yield).

¹H NMR (CDCl₃, 500 MHz, TMS): δ 8.59 (d, *J* = 2.0 Hz, 1H), 8.43 (dd, *J₁* = 9.0 Hz, *J₂* = 2.0 Hz, 1H), 7.83 (d, *J =* 9.0 Hz, 1H), 7.37-7.28 (m, 5H), 4.52-4.49 (dd, *J₁ =* 9.5 Hz, *J₂* = 4.5 Hz, 1H), 3.24 (dd, *J₁ =* 14 Hz, *J₂* = 4.5 Hz, 1H), 3.11 (dd, *J₁ =* 14 Hz, *J₂* = 9.5 Hz, 1H).

### Example 12 Preparation of 1-(4-nitro-2-(trifluoromethyl)phenyl)ethan-1-one

To a 50 mL reaction flask were sequentially added 1.1 g of potassium carbonate, 2.2 g of water, 1 g of 2-(4-nitro-2-(trifluoromethyl)phenyl)propionitrile, 0.1 g of copper(I) chloride and 10 mL of *N,N-*dimethylformamide, and the atmosphere in the reaction flask was replaced with oxygen atmosphere. The reaction mixture was heated in a 60°C oil bath, reacted at 60°C for 2 h, cooled and added into 50 mL of methylbenzene. The resultant reaction solution was washed, concentrated and purified to obtain 0.9 g of 1-(4-nitro-2-(trifluoromethyl)phenyl)ethan-1-one (94% yield).

¹H NMR (CDCl₃, 500 MHz, TMS): δ 8.58 (d, *J* = 1.5 Hz, 1H), 8.48 (dd, *J₁* = 8.5 Hz, *J₂* = 1.5 Hz, 1H), 7.66 (d, *J =* 8.5 Hz, 1H), 2.63 (s, 3H). ¹³C NMR (CDCl₃, 125 MHz): δ 199.8, 148.2, 145.7, 128.5 (q, *J* = 33 Hz), 128.4, 126.9, 122.3 (q, *J* = 273 Hz), 122.3 (q, *J* = 6 Hz), 30.5.

### Example 13 Preparation of 1-(4-(4-chlorophenoxy)-2-(trifluoromethyl)phenyl)ethan-1-one

To a 25 mL reaction flask were sequentially added 0.5 g of 1-(4-nitro-2-(trifluoromethyl)phenyl)ethan-1-one, 0.3 g of 4-chlorophenol, 0.2 g of potassium carbonate and 2.5 mL of *N,N-*dimethylformamide. The reaction mixture was heated in a 125°C oil bath, reacted at 125°C for 5 h, cooled and added into 50 mL of methylbenzene. The resultant reaction solution was washed, concentrated and purified to obtain 0.57 g of 1-(4-(4-chlorophenoxy)-2-(trifluoromethyl)phenyl)ethan-1-one (85% yield).

### Example 14 Preparation of mefentrifluconazole

To a 50 mL three-necked flask were sequentially added 0.7 g of water and 3.9 g of dimethyl sulfate. The reaction mixture was heated to 33°C, dropwise added with 2.0 g of dimethyl sulfide, stirred for 15 min and added with 6.3 g of 1-(4-nitro-2-(trifluoromethyl)phenyl)ethan-1-one prepared in Example 12 at 35°C. Then, the reaction mixture was added with 4.5 g of a 85 wt.% potassium hydroxide solution at 35-45°C under stirring, and stirred at 38°C for 2 h until raw materials were confirmed to be completely consumed. After that, the reaction mixture was added with 30 g of water at 60°C, and stirred for 20 min, and an organic phase was collected. The organic phase was dissolved in 30 g of dimethyl formamide (DMF), and subjected to distillation to remove dimethyl sulfide to obtain a DMF solution of 2-[4-(4-chlorophenoxy)-2-(trifluoromethyl) phenyl]-2-methyloxiran, which was further analyzed by high performance liquid chromatography (HPLC). The results demonstrated that 6.6 g of the product of interest (99% yield) was obtained.

The DMF (30 g) solution of 2-[4-(4-chlorophenoxy)-2-(trifluoromethyl) phenyl]-2-methyloxiran (6.6 g) was heated to 60°C, sequentially added with 1.7 g of 99 wt.% 1,2,4-triazole and 0.3 g of sodium hydroxide powder under stirring, heated to 125°C and reacted at 125°C under stirring for 5 h until raw materials were confirmed to be completely consumed. The reaction mixture was evaporated under reduced pressure to remove most of the DMF, added with 30 g of methylbenzene and 20 g of water, and subjected to separation at 60°C to collect a methylbenzene phase. The methylbenzene phase was washed with 20 g of water, and subjected to separation to collect a methylbenzene solution. The methylbenzene solution was concentrated under reduced pressure to obtain a solution containing about 50 wt.% of the desired product. The solution was heated (about 80°C) for dissolution, slowly cooled to 0°C under stirring, stirred at 0°C for 30 min, and filtered under vacuum to collect a filter cake. The filter cake was washed twice with 10 g of methylbenzene which was pre-cooled to 0°C, and dried to obtain 6.6 g of mefentrifluconazole (84% yield).

Described above are merely illustrative of the disclosure, and are not intended to limit the disclosure. Although the disclosure has been illustrated and described in detail above, it should be understood that those skilled in the art could still make modifications and replacements content to the embodiments content of the disclosure. Those modifications and replacements content made by those skilled in the art based on the content disclosed herein without departing from the scope of the disclosure shall fall within the scope of the present disclosure defined by the appended claims.

## Claims

1. A method for preparing a compound of formula (1), comprising:
reacting a compound (2) with a compound (3) in the presence of a base to obtain the compound (1), as shown in the following reaction scheme: 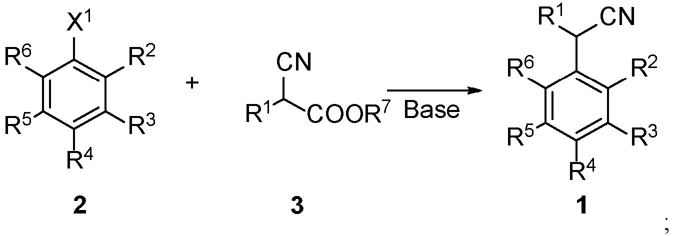
wherein R¹ is a carbon-based group;
R², R³, R⁴, R⁵, and R⁶ are independently selected from the group consisting of hydrogen, C₁-C₁₀ alkyl, C₆-C₁₂ aryl, a heteroaryl group containing one or two atoms selected from nitrogen, oxygen and sulfur, halogen, nitro, a cyano group, a C₁-C₆ alkyl-acyl group, a C₁-C₆ alkoxy group, a C₆-C₁₂ aryloxy group and -COOR⁸, wherein the alkyl, the aryl and the heteroaryl group are unsubstituted or substituted with halogen; or, any two adjacent groups of R², R³, R⁴, R⁵, and R⁶ together form a C₁-C₁₀ cyclic substituent; the C₁-C₁₀ cyclic substituent is free of heteroatoms or containing one or two atoms selected from nitrogen, oxygen and sulfur; and the cyclic substituent is unsubstituted or substituted with halogen;
at least one of R², R⁴ and R⁶ is selected from the group consisting of a C₁-C₄ haloalkyl group, nitro, a cyano group, halogen, a C₁-C₆ alkyl-acyl group and -COOR⁸; wherein R⁸ is a carbon-based group;
R⁷ is selected from the group consisting of hydrogen, C₁-C₁₀ alkyl, C₆-C₁₂ aryl, and a heteroaryl group containing one or two atoms selected from nitrogen, oxygen and sulfur; and the alkyl, the aryl and the heteroaryl group is unsubstituted or substituted by halogen;
X¹ is halogen or -OSO₂R⁹; wherein R⁹ is selected from the group consisting of C₁-C₁₀ alkyl, C₆-C₁₂ aryl, and a heteroaryl group containing one or two atoms selected from nitrogen, oxygen and sulfur, wherein the alkyl, the aryl and the heteroaryl group is unsubstituted or substituted by halogen; and
the base is selected from the group consisting of an alkali metal carbonate, an alkali metal bicarbonate, an alkali metal phosphate, an alkali metal C₁-C₆ alkyl carboxylate, an alkali metal formate, an alkali metal hydroxide, an alkali metal alcoholate, an alkali metal hydride, an alkaline earth metal carbonate, an alkaline earth metal bicarbonate, an alkaline earth metal phosphate, an alkaline earth metal C₁-C₆ alkyl carboxylate, an alkaline earth metal formate, an alkaline earth metal hydroxide, an alkaline earth metal alcoholate, an alkaline earth metal hydride, an alkali metal alkylide, an alkali metal amide, an organic amine compound, and a combination thereof.

2. The method according to claim 1, **characterized in that** the base is selected from the group consisting of an alkali metal carbonate, an alkali metal bicarbonate, an alkali metal phosphate, an alkali metal hydroxide, an alkali metal C₁-C₆ alkyl carboxylate, an alkali metal formate, a tertiary amine compound, and a combination thereof.

3. The method according to claim 2, **characterized in that** the base is selected from the group consisting of potassium carbonate, potassium hydroxide, sodium carbonate, sodium hydroxide and a tertiary amine compound; wherein the tertiary amine compound is an alkyl tertiary amine compound.

4. The method according to any one of claims 1-3, **characterized in that** R¹ is selected from the group consisting of C₁-C₁₀ alkyl, C₆-C₁₂ aryl, and a heteroaryl group containing one or two atoms selected from nitrogen, oxygen and sulfur; wherein the alkyl, the aryl and the heteroaryl group are unsubstituted or substituted by halogen;
R² and R⁴ are independently selected from the group consisting of a C₁-C₄ haloalkyl group, nitro, a cyano group, a C₂-C₄ alkyl-acyl group and -COOR⁸; wherein R⁸ is C₁-C₄ alkyl or a C₁-C₂ haloalkyl group; and
R³, R⁵, and R⁶ are independently selected from the group consisting of hydrogen and C₁-C₄ alkyl.

5. The method according to claim 4, **characterized in that** R¹ is C₁-C₄ alkyl; R² is trifluoromethyl; R³, R⁵, and R⁶ are independently hydrogen; and R⁴ is nitro; and
X¹ is fluorine, chlorine, bromine or iodine.

6. The method according to claim 5, **characterized in that** X¹ is chlorine.

7. The method according to claim 1, **characterized in that** a molar ratio of the compound (3) to the compound (2) is (0.7-3.5):1; and
a molar ratio of the compound (3) to the base is 1:(0.3-2.0).

8. The method according to claim 7, **characterized in that** the molar ratio of the compound (3) to the compound (2) is (1.4-2.2): 1; and
the molar ratio of the compound (3) to the base is 1:(0.7-2.0).

9. The method according to claim 1, **characterized in that** a molar ratio of the compound (3) to the compound (2) is (1.6-1.8):1; and
a molar ratio of the compound (3) to the base is 1:(0.9-1.1).

10. A use of the method according to claim 1 in the preparation of a triazole fungicide, **characterized in that** the triazole fungicide is selected from the group consisting of mefentrifluconazole, Ipfentrifluconazole, epoxiconazole, flutriafol, cyproconazole, bromuconazole, difenoconazole, propiconazole, etaconazole and hexaconazole.
